Europäisches Patentamt

European Patent Office

Office européen des brevets

(19)

(11) Veröffentlichungsnummer : **0 066 582**
**B1**

(12) # EUROPÄISCHE PATENTSCHRIFT

(45) Veröffentlichungstag der Patentschrift :
02.05.84

(21) Anmeldenummer : 81903250.9

(22) Anmeldetag : 04.12.81

(86) Internationale Anmeldenummer :
PCT/CH 81/00137

(87) Internationale Veröffentlichungsnummer :
WO WO/82020 (24.06.82 Gazettee 82/16)

(51) Int. Cl.³ : **C 12 M 1/00, C 12 P 5/02**

(54) **VERFAHREN UND ANLAGE ZUR VERWERTUNG VON HAUSMÜLL UND ANDERN ORGANISCHEN ABFÄLLEN ZUR GEWINNUNG VON METHANGAS.**

(30) Priorität : 12.12.80 CH 9215/80

(43) Veröffentlichungstag der Anmeldung :
15.12.82 Patentblatt 82/50

(45) Bekanntmachung des Hinweises auf die Patenterteilung : 02.05.84 Patentblatt 84/18

(84) Benannte Vertragsstaaten :
FR

(56) Entgegenhaltungen :
Keine

(73) Patentinhaber : HUNZIKER, Martin
Speerstrasse 50
.CH-8030 Zürich (CH)

SCHILDKNECHT, Albert
Dorfstrasse 45
CH-8955 Oetwil a. d. Limmat (CH)

(72) Erfinder : HUNZIKER, Martin
Speerstrasse 50
CH-8030 Zürich (CH)
Erfinder : SCHILDKNECHT, Albert
Dorfstrasse 45
CH-8955 Oetwil a. d. Limmat (CH)

(74) Vertreter : Meyer, Reinhard
c/o EGLI PATENTANWÄLTE Horneggstrasse 4
CH-8008 Zürich (CH)

Verfahren und Anlage zur Verwertung von Hausmüll und andern organischen Abfällen zur Gewinnung von Methangas

Die Erfindung betrifft ein Verfahren zur Verwertung von Hausmüll und andern, organische Stoffe enthaltenden Abfällen durch Abbau dieser Stoffe in Reaktionsräumen unter Erzeugung von Methangas, wobei die Stoffe in aeroben und anaeroben Phasen geführt werden, und eine Anlage zur Durchführung des Verfahrens.

Die durch Mensch und Tier verursachten Abfallstoffe werden in bekannten Verfahren beseitigt, wobei vor allem die Kehrichtverbrennung und die Kompostierung Anwendung finden. In Kläranlagen werden zudem das verunreinigte Wasser und Fäkalien behandelt, wobei der dort anfallende Schlamm getrocknet und entweder als Dünger verwendet oder verbrannt wird. Es ist auch bekannt, den in den Kläranlagen anfallenden Schlamm durch methanerzeugende Bakterien abzubauen, wobei das hierbei entstehende Methangas für die Erzeugung von Energie und Wärme verwendet wird. Die bisher bekannten Verfahren sind zwar verhältnismässig wirkungsvoll, doch haftet ihnen der Nachteil an, dass die Zeit für den bakteriellen Abbau der organischen Stoffe zu lang dauert. Dies bedingt die Errichtung von in bezug auf die zu verarbeitenden Abfallmengen grossdimensionierten Behandlungsanlagen.

Bekannte Abbauverfahren (FR-A-893 537 und FR-A-1 107 499), bei denen der Schlamm unter Erzeugung von Methangas mit mesophilen Bakterien (bis etwa 27 °C) abgebaut wird, benötigen wegen der verhältnismässig niederen Betriebstemperatur lange Behandlungszeit, weshalb grossflächige Anlagen und damit entsprechend grosse Aufwendungen erforderlich sind.

Bei einem bekannten kontinuierlichen Abbauverfahren mit thermophilen Bakterien ist es bekannt (US-A-4 213 857), den aus den Abfällen gebildeten Schlamm zuerst in einem separaten Kessel bei 55-75 °C vorzubehandeln. Danach wird der Schlamm einem Separator zugeführt, wo die Feststoffe abgetrennt werden. Die flüssige Phase wird dann der anaeroben Stufe zugeführt. Der hierfür benötigte Wärmebedarf ist jedoch wegen der niedrigen Ausgangstemperatur des Schlammes und wegen der bis zum Beginn der anaeroben Behandlungsphase unvermeidlichen Wärmeverluste beträchtlich. Erfolgt jedoch die Aufheizung des Schlammes unmittelbar in den Abbaubehältern (US-A-4 022 665, FR-A-2 464 297, FR-A-2 466 502 und FR-A-2 481 874), müssen zur Vermeidung einer Verlängerung der Verweildauer infolge langer Aufheizzeiten leistungsfähige und dadurch aufwendige Heizeinrichtungen installiert werden.

Beim Chargenbetrieb (FR-A-898 669 und FR-A-1 009 247), bei dem die Abfälle chargenweise in Behältern abgebaut werden, muss die Wärmezufuhr ebenfalls zu den Behältern erfolgen, entweder durch Beheizen einer in die Behälter eingeleiteten anaeroben Flüssigkeit oder durch Beheizung im Behälter selbst. Auch hier verlängert das Aufheizen im Behälter die Verweilzeit des Schlammes. Zur Verminderung des Heizaufwandes bei Abbauanlagen ist es bekannt (US-A-4 057 401), Wärme durch Sonnenenergie zu erzeugen und diese in die Abbaubehälter zu leiten. Aber auch in diesem Falle ist eine Erhöhung der Verweilzeit wegen der Aufheizzeit nicht zu vermeiden.

Der Erfindung liegt die Aufgabe zugrunde, ein Verfahren zur Gewinnung von Methangas der eingangs beschriebenen Art so auszugestalten, dass der bakterielle Abbau der organischen Abfälle durch Vermeidung oder wenigstens durch wesentliche Verringerung zusätzlicher Heizzeiten beschleunigt, ohne dadurch den Abbauvorgang in ungünstiger Weise zu beeintrachtigen, und gleichzeitig der Wärmebedarf verringert werden kann.

Diese Aufgabe wird gemäss der Erfindung dadurch gelöst, dass in der anaeroben Phase den in fliessfähigem Zustand übergeführten Stoffen vor dem Eintritt in die Reaktionsräume Wärme zugeführt wird.

Zur Durchführung des erfindungsgemässen Verfahrens dient eine Anlage, bei der erfindungsgemäss der der anaeroben Phase dienende Anlageteil einen Wartebehälter, mindestens einen Reaktionsbehälter und eine zwischen dem Warte- und dem Reaktionsbehälter liegende Heizvorrichtung für das Erwärmen der Stoffe aufweist.

Die Erfindung ist in der Zeichnung in einem Ausführungsbeispiel dargestellt und nachfolgend beschrieben. Es zeigen :

Figur 1  eine schematische Darstellung einer erfindungsgemässen Anlage im Aufriss und

Figur 2  einen Grundriss der Anlage nach Fig. 1.

Die Erfindung geht von der Ueberlegung aus, dass eine Beschleunigung des bakteriellen Abbaus organischer Abfälle durch thermophile Bakterien, d. h. bei einer Abbautemperatur von 50-60 °C, nur durch zusätzliche Massnahmen voll ausgenützt werden kann. Einerseits bedingt dies die Zuführung von Wärme zur Erwärmung der organischen Stoffe, andererseits soll jedoch diese Wärmezufuhr derart erfolgen, dass eine dadurch bedingte Verlängerung der Verweilzeit vermieden wird und zudem ein Teil des Wärmebedarfs bereits bei der Vorbereitung des Schlamms zu Beginn der anaeroben Phase durch bakterielle Abbautätigkeit entsteht.

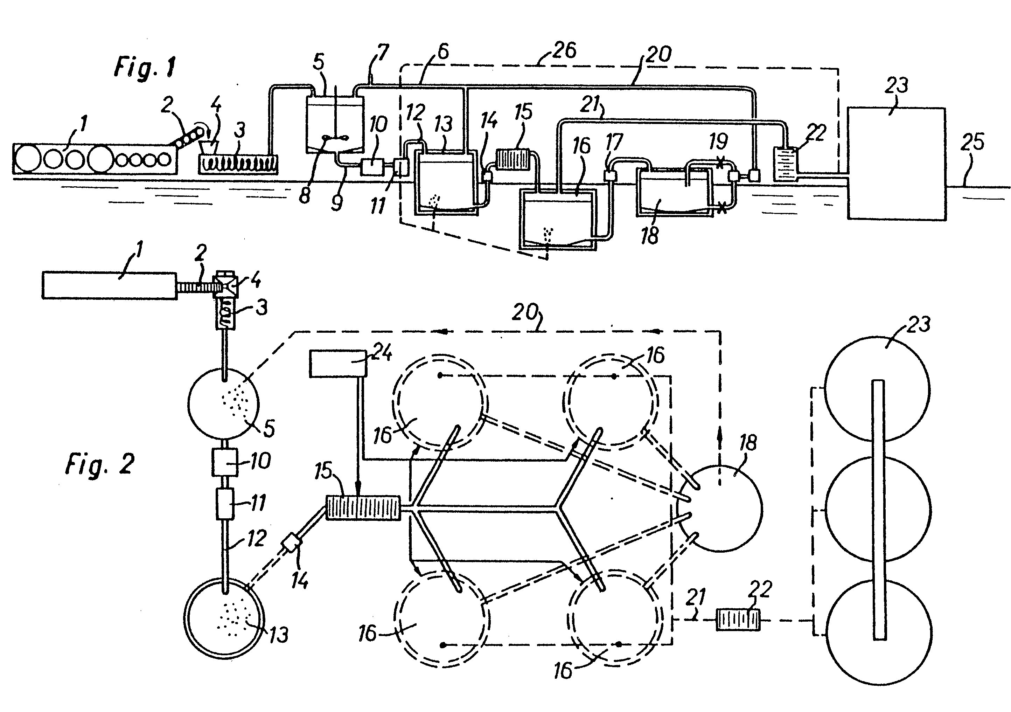

Die in Fig. 1 und 2 dargestellte Anlage dient der Verwertung von Hausmüll, wobei jedoch die aus dem Hausmüll aussortierten organischen Stoffe mit andern organischen Abfällen, z. B. Mist, Jauche, Klärschlamm o. dgl., gemischt werden können.

Der in die Anlage ankommende Hausmüll wird zunächst einer schematisch dargestellten Sortieranlage 1 zugeleitet, in der die organischen Stoffe von den übrigen Materialien, wie z. B. Metalle, Glas, Kunststoffe, Kartons, getrennt werden. Die organischen Stoffe werden durch einen Förderer 2 einer Dosiervorrichtung 3 zugeführt. Die Dosiervorrichtung 3, z. B. eine Dosierschnecke, weist einen auf ihrer Oberseite angeordneten, leicht konisch ausgebildeten Einfüllschacht 4 auf. Vom Ausgang der Dosierschnecke 3 werden die organischen Stoffe in einen Mischbehälter 5 gefördert. In den Mischbehälter 5 wird auch Flüssigkeit über eine Flüssigkeitszuleitung 6 geleitet. Die Flüssigkeitszuleitung 6 weist einen Anschluss 7 auf, durch den zusätzlich organische Materialien in den Mischbehälter 5 eingeführt werden können, z. B. Jauche oder Klärschlamm. Der Inhalt des Mischbehälters 5 wird durch ein schematisch dargestelltes mechanisches Rührwerk 8 gemischt. Der Mischbehälter 5 weist keine besonderen Vorrichtungen zur Fernhaltung von Sauerstoff auf ; es herrschen darin aerobe Verhältnisse.

Am Boden des Mischbehälters 5, der zweckmässig zur Mitte geneigt ist, führt eine Leitung 9 zu einer Zerkleinerungsvorrichtung, die aus einem Grobzerkleinerer 10 und einem nachgeschalteten Feinzerkleinerer 11 besteht. Die Zerkleinerer 10, 11 sind zweckmässig Mühlen, z. B. Scheibenmühlen, wobei die Feinmühle zwecks Homogenisierung des sich bildenden Schlamms eine Zerkleinerung bis zu einer Teilchengrösse von etwa 1,5 mm erreicht. Die zerkleinerten organischen Stoffe werden über eine Verbindungsleitung 12 in einen Wartebehälter 13 übergeleitet. Der Wartebehälter 13 ist luftdicht abgeschlossen. Der darin befindliche Schlamm wird durch Methangas, das unter Druck durch im Boden des Wartebehälters 13 befindliche Mündungen gepresst wird, gerührt. Es entsteht ein sauerstofffreies Gemisch von Schlamm und Methangas, das durch eine Pumpe 14 durch einen Wärmeaustauscher in einen Reaktionsbehälter 16 gefördert wird. Da im Wartebehälter 13 anaerobe Verhältnisse herrschen, beginnt hier bereits der Abbau der organischen Stoffe durch anaerobe Bakterien, wodurch die Temperatur des Schlamms ansteigt, und zwar bis etwa 30 °C. Nach dem Durchgang durch den Wärmeaustauscher 15, der z. B. als Röhrenwärmeaustauscher ausgebildet sein kann, erreicht der Schlamm eine Temperatur von etwa 60 °C. Bei dieser Temperatur vermehren sich die thermophilen Bakterien und bauen die organischen Stoffe mit erhöhter Intensität ab. Es entsteht hierbei ein Gemisch von Kohlendioxid und Methangas, etwa im Verhältnis von 35 Vol.-% Kohlendioxid und 65 % Methangas. Auch im Reaktionsbehälter 16 wird eine intensive Rührbewegung des Schlammes aufrechterhalten, was wie beim Wartebehälter 13 durch Einpressen von Methangas erfolgt.

Im Reaktionsbehälter 16 werden die organischen Stoffe bis auf etwa 80 % abgebaut. Der im Reaktionsbehälter 16 verbleibende Restschlamm wird durch eine Pumpe 17 in ein Restschlamm-Bassin 18 gefördert. Dort wird die Flüssigkeit von den restlichen Stoffen getrennt und durch eine Pumpe 19 über eine Förderleitung 20 je nach Bedarf zum Mischbehälter 5 und/oder Wartebehälter 13 gefördert, um die gewünschte Zusammensetzung des homogenisierten Schlammes zu erreichen. Dieser Schlamm weist eine Trockensubstanz von etwa 6 % auf, jedoch kann dieser Anteil je nach den jeweiligen Verhältnissen erhöht werden. Wesentlich ist, dass im Reaktionsbehälter 16 die Temperatur auf etwa 60 °C gehalten werden kann. Zu diesem Zweck werden im Reaktionsbehälter 16 Heizschlangen angeordnet und durch einen Wärmeträger beaufschlagt.

Das im Reaktionsbehälter 16 anfallende Gas wird über eine Leitung 21 in eine Trennanlage 22 gefördert und dort in seine beiden Komponenten Kohlendioxid und Methangas zerlegt, worauf das Methangas in Gasometern 23 gespeichert wird. Das Methangas wird teilweise für die Rührbewegung des Schlammes im Wartebehälter 13 und im Reaktionsbehälter 16 verwendet. Mit dem verbleibenden Methangas kann ein Gasmotor (nicht dargestellt) betrieben werden, der einen Elektro-Generator treibt. Dieser liefert die elektrische Energie zum Antrieb elektrischer Motoren, die beispielsweise in der Dosiervorrichtung 3, beim Rührwerk 8, bei den Mühlen 10, 11, bei den Pumpen 14, 17, 19, beim Förderer 2 und bei der Dosierschnecke 3 eingebaut sind. Bei zweckmässiger Auslegung der beschriebenen Anlage bleibt noch Methangas übrig, welches z. B. für die Wärmeerzeugung verwendet werden kann, zu der auch die Abwärme des erwähnten Gasmotors herangezogen werden kann. Es lässt sich somit ein autarker Betrieb der Anlage durchführen, dies obwohl durch den Einsatz thermophiler Bakterien und dementsprechend der Erhöhung der Abbautemperatur die Abbaugeschwindigkeit wesentlich beschleunigt wird.

Das beschriebene Verfahren verläuft demnach sowohl in aeroben als auch in anaeroben Phasen. Die aerobe Phase unterteilt sich in eine aerobe Vorphase mit den Anlageteilen Sortieranlage 1, Förderer 2, Dosierschnecke 3, Mischbehälter 5, Zerkleinerungsvorrichtung 10, 11 und in eine aerobe Nachphase mit den Anlageteilen Restschlamm-Bassin 18, Pumpe 19 und Leitung 20. Die anaerobe Phase umfasst den Wartebehälter 13 mit einer Temperaturstufe von 30 °C, den Wärmeaustauscher 15, den Reaktionsbehälter 16 und die Pumpe 17.

Aus Fig. 2 sind die anlageinternen Verbindungen etwas deutlicher ersichtlich. Der Weg des Schlammes ist durch ausgezogene und gestrichelte Doppellinien dargestellt. Der Transport des Methangases und des Kohlendioxids aus den Reaktionsbehältern 16 ist durch eine gestrichelte Linie dargestellt. Die Rückleitung des Wassers aus dem Restschlamm im Restschlamm-Bassin 18 erfolgt über die punktierte Leitung 20. Die Wärmezufuhr aus einer Wärmeerzeugungsanlage 24 zum Wärmeaustauscher 15 und in die einzelnen Reaktionsbehälter 16 ist durch ausgezogene einfache Linien dargestellt. Fig. 2 zeigt auch die Zahl der einzelnen Anlageteile, die für die beschriebene Anlage erforderlich sind, während in Fig. 1 die Lage der Anlageteile gegenüber dem Boden ersichtlich ist. Die Sortieranlage 1, die Dosierschnecke 3, der Mischbehälter 5 und die Zerkleinerungsvorrichtung 10, 11 befinden sich oberhalb des Bodens 25 und sind zudem in einem nicht dargestellten Gebäude untergebracht. Der Mischbehälter 5 ist teilweise im Boden 25 eingebettet, während der bzw. die Reaktionsbehälter 16 ganz im Boden 25 liegen und zudem eine wirkungsvolle Isolation aufweisen. Das Restschlamm-Bassin 18 liegt teilweise im Boden 25, während die Pumpe 19, die Trennanlage 22 und mindestens teilweise auch die Gasometer 23 über dem Bodem 25 angeordnet sind.

Anhand von Fig. 1 und 2 soll ein Beispiel für eine solche Anlage erläutert werden. Für eine Anlage, die pro Tag 27 t frischen, nichtsortierten Hausmülls, entsprechend 15 t abbaufähiger Stoffe, verarbeitet, ist ein Mischbehälter 5 von etwa 400-500 m³, d. h. etwa 30 m³ pro t, erforderlich. Zweckmässig ist der Mischbehälter 5 zylindrisch, um mit dem Rührwerk 8 eine gute Rührwirkung zu erreichen. Die Abfälle verbleiben etwa 24 Stunden im Mischbehälter 5.

Nach der Zerkleinerungsvorrichtung 10, 11 werden die gemahlenen Stoffe, denen Flüssigkeit aus dem Restschlamm-Bassin 18 zugesetzt wird, bis eine Mischung mit 5-6 Gew.% Trockensubstanz erreicht wird, in den Wartebehälter, ebenfalls mit etwa 400-500 m³ Inhalt, übergeführt. Der Wartebehälter 13 ist vollständig geschlossen, und der Schlamm wird durch eingeführtes Methangas, das nach der Trennanlage 22 entnommen und in einer Leitung 26, siehe Fig. 1, zugeleitet wird, in ständiger Bewegung gehalten. Der Wartebehälter 13 weist zweckmässig ebenfalls zylindrische Form mit konisch geneigtem Boden auf ; der Schlamm verweilt hier ebenfalls etwa 24 Stunden.

Für die eigentliche Methangärung einer Temperatur von 55-60 °C werden, siehe Fig. 2, vier Reaktionsbehälter 16 mit einem Inhalt von 500-600 m³ benötigt, um die genannte Menge von 15 t abbaufähiger Stoffe zu verarbeiten. In den Reaktionsbehältern 16 verbleibt der Schlamm während etwa 8 Tagen. Während dieser Zeit ist es erforderlich, täglich eine bestimmte Menge Schlamm abzuleiten und neuen Schlamm zuzuführen, um eine konstante Aktivität ohne Erschöpfung oder Sättigung aufrechterhalten zu können. Bei den Reaktionsbehältern 16 handelt es sich um geschlossene Behälter, die ebenfalls zylindrische Form mit konischem Boden aufweisen können.

Das entstehende Gas weist etwa 65 % Methan ($CH_4$) und 35 % Kohlendioxid ($CO_2$) auf. Pro Tonne aussortierten Hausmülls können etwa 140 Nm³ Methangas erzeugt werden, das bei der anfallenden Menge von 15 t pro Tag 2 175 Nm³ ergibt. Das anfallende Gas weist einen hohen Wärmewert, etwa 40 600 kJ/Nm³ entsprechend 9 700 kcal, auf und ist demnach gut verwendbar. Am Ausgang der Gasometer 23 ist es zudem verhältnismässig rein.

Der im Restschlamm-Bassin 18 anfallende Restschlamm enthält noch etwa 20 % der Stoffe, was bei 15 t täglichem Anfall etwa 3 t Kompost mit einem Flüssigkeitsgehalt von etwa 53 % entspricht.

Selbstverständlich müssen die Anlageteile nicht immer genau nach Fig. 2 angeordnet sein, z. B. können die Reaktionsbehälter 16 auch nur teilweise im Boden 25 eingebettet sein.

Der Behälter 18 kann aber auch zur Pasteurisierung des Restschlamms verwendet oder zu zusätzlichen Erzeugung von Methangas aus dem im Behälter 18 anfallenden Restschlamm ausgenützt werden. Im letzteren Fall wird die Nachphase anaerob geführt.

**Ansprüche**

1. Verfahren zur Verwertung von Hausmüll und andern, organische Stoffe enthaltenden Abfällen durch Abbau dieser Stoffe in Reaktionsräumen unter Erzeugung von Methangas, wobei die Stoffe in aerober und anaerober Phase geführt werden, dadurch gekennzeichnet, dass in der anaeroben Phase den in fliessfähigem Zustand übergeführten Stoffen vor dem Eintritt in die Reaktionsräume Wärme zugeführt wird.

2. Verfahren nach Anspruch 1, dadurch gekennzeichnet, dass die Stoffe auf 55-60 °C erwärmt und in den Reaktionsräumen auf dieser Temperatur, z. B. durch weitere Wärmezufuhr, zwecks Bildung thermophiler Bakterien gehalten werden.

3. Verfahren nach Anspruch 1 oder 2, dadurch gekennzeichnet, dass die Stoffe vor dem Eintritt in die anaerobe Phase in einer aeroben Vorphase zerkleinert werden, vorzugsweise durch eine Grob- und eine Feinzerkleinerung.

4. Verfahren nach Anspruch 3, dadurch gekennzeichnet, dass die Stoffe durch Zugabe von Flüssigkeit, z. B. aus dem aus den Reaktionsräumen anfallenden Restschlamm, in einen Schlamm mit 5-10 Gew.% Trockensubstanz übergeführt werden.

5. Verfahren nach Anspruch 3, dadurch gekennzeichnet, dass die Stoffe vor ihrer Zerkleinerung dosiert und mit Flüssigkeit gemischt werden.

6. Anlage zur Durchführung des Verfahrens nach Anspruch 1, dadurch gekennzeichnet, dass der der anaeroben Phase dienende Anlageteil einen Wartebehälter (13), mindestens einen Reaktionsbehälter (16) und eine zwichen dem Warte- und dem Reaktionsbehälter liegende Heizvorrichtung (15) für das Erwärmen der Stoffe aufweist.

7. Anlage nach Anspruch 6, dadurch gekennzeichnet, dass die Heizvorrichtung (15) ein Wärmeaustauscher, z. B. ein Röhrenwärmeaustauscher, ist.

8. Anlage nach Anspruch 7, dadurch gekennzeichnet, dass der der aeroben Vorphase dienende Anlageteil einen Mischbehälter (5) zur Aufnahme der durch eine Dosiervorrichtung (3) dosierten Stoffe und von Flüssigkeit und eine Zerkleinerungsvorrichtung, z. B. eine Grobmühle (10) und eine Feinmühle (11), aufweist.

9. Anlage nach Anspruch 7 oder 8, dadurch gekennzeichnet, dass der z. B. der aeroben Nachphase dienende Anlageteil einen dem Reaktionsbehälter bzw. den Reaktionsbehältern (16) nachgeordneten Restschlamm-Behälter (18) aufweist.

10. Anlage nach Anspruch 9, dadurch gekennzeichnet, dass der Behälter (18) für anaerobe Behandlung oder zur Pasteurisierung des Restschlamms ausgebildet ist.

## Claims

1. Process for the utilization of household rubbish or garbage and other waste products containing organic substances by decomposing these substances in reaction chambers, accompanied by the production of methane gas, the decomposition of the substances being conducted in aerobic and anaerobic phases, characterized in that in the anaerobic phase, heat is supplied to the substances brought into the flowable state prior to entering the reaction chambers.

2. Process according to claim 1, characterized in that the substances are heated to 55 to 60 °C and are maintained in the reaction chambers at this temperature, e. g. through further heat supply, for the formation of thermophilic bacteria.

3. Process according to claims 1 or 2, characterized in that prior to entering the anaerobic phase, the substances are crushed in an aerobic pre-phase, preferably by coarse and fine crushing.

4. Process according to claim 3, characterized in that by adding liquid, e. g. the residual sludge obtained from the reaction chambers, the substances are converted into a sludge with a 5 to 10 % by weight dry substances content.

5. Process according to claim 3, characterized in that prior to crushing the substances are proportioned and mixed with liquid.

6. Plant for performing the process according to claim 1, characterized in that the plant part used in the anaerobic phase comprises a waiting container (13), at least one reaction vessel (16) and a heating means (15) for heating the substances located between the waiting container and the reaction vessel.

7. Plant according to claim 6, characterized in that the heating means (15) is a heat exchanger, e. g. tubular exchanger.

8. Plant according to claim 7, characterized in that the plant part used for the aerobic pre-phase comprises a mixing tank (5) for receiving the substances proportioned by a proportioning means (3) and liquid and a crushing means, e. g. a coarse mill (10) and a fine mill (11).

9. Plant according to claims 7 or 8, characterized in that the plant part used e. g. for the aerobic postphase has a residual sludge tank (18) following the reaction vessel or vessels (16).

10. Plant according to claim 9, characterized in that the tank (18) is constructed for anaerobic treatment or pasteurization of the residual sludge.

## Revendications

1. Procédé pour la récupération d'ordures ménagères et d'autres déchets contenant des matières organiques par la décomposition de ces matières dans des espaces de réaction avec la production de méthane, les matières étant alors conduites dans des phases aérobies et anaérobies caractérisé en ce que de la chaleur est amenée pendant la phase anaérobie aux matières à l'état liquide avant l'entrée dans l'espace de réaction.

2. Procédé selon la revendication 1, caractérisé en ce que les matières sont réchauffées à 55 ou 60 °C et en ce qu'elles sont maintenues à cette température dans les espaces de réaction (16), par exemple, par l'apport de chaleur afin de former des bactéries thermophiles.

3. Procédé selon les revendications 1 ou 2, caractérisé en ce que les matières sont broyées pendant une pré-phase aérobie avant de pénétrer dans la phase anaérobie, de préférence par un broyage grossier et fin.

4. Procédé selon la revendication 3, caractérisé en ce que les matières sont transportées sous forme de boue présentant une teneur en matière sèche de 5 à 10 % en poids en additionnant du liquide provenant par exemple, de la boue restante du ou des espaces de réaction (16).

5. Procédé selon la revendication 3, caractérisé en ce que les matières sont dosées et mélangées avec un liquide avant d'être broyées.

6. Installation pour réaliser le procédé selon la revendication 1, caractérisée en ce que la partie servant à la phase anaérobie présente un récipient d'attente (13), au moins un récipient de réaction (16) et, entre les récipients d'attente et de réaction, un dispositif de chauffage (15) pour réchauffer la matière.

7. Installation selon la revendication 6, caractérisée en ce que le dispositif de chauffage (15) est réalisé par un échangeur de chaleur, par exemple un échangeur de chaleur tubulaire.

8. Installation selon la revendication 7, caractérisée en ce que la partie servant à la pré-phase aérobie de l'installation présente un récipient de mélange (5) pour recevoir la matière dosée par le dispositif de dosage (3) et du liquide, et un dispositif de broyage, par exemple un broyeur grossier (10) et un broyeur fin (11).

9. Installation selon les revendications 7 ou 8, caractérisée en ce que la partie de l'installation servant à la phase ultérieure aérobie présente un bassin de boue restante (18) mis en place après le récipient de réaction ou les récipients de réaction (16).

10. Installation selon la revendication 9, caractérisée en ce que le récipient (18) est réalisé pour un traitement anaérobie ou pour pasteuriser la boue restante.

*Fig. 1*

*Fig. 2*

0 066 582